# EUROPÄISCHE PATENTSCHRIFT

(11) **EP 0 913 130 B1**
(45) Veröffentlichungstag und Bekanntmachung des Hinweises auf die Patenterteilung: **12.03.2003**
(21) Anmeldenummer: 98115809.0
(22) Anmeldetag: 21.08.1998
(51) Int. Cl.: A61C 13/00, A61K 6/02, A61K 6/027, A61K 6/087

(54) **Verfahren und Vorrichtung zur Herstellung eines Zahnersatzteiles**
Method and device for manufacturing a dental prosthesis
Méthode et dispositif pour la fabrication d'une prothèse dentaire

(30) Priorität: 31.10.1997 CH 253097
(43) Veröffentlichungstag der Anmeldung: 06.05.1999
(73) Patentinhaber: DCS Forschungs & Entwicklungs AG, 4123 Allschwil (CH)
(72) Erfinder: Guiot, Jean-Bernard, 68200 Mulhousen (FR); Traber, Tony, 4123 Allschwil (CH); Efroni, Eran, 4055 Basel (CH)
(74) Vertreter: Hotz, Klaus, Dipl.-El.-Ing./ETH

(56) Entgegenhaltungen:
- EP-A- 0 634 150
- WO-A-96/10371
- DE-A- 3 213 014
- DE-U- 9 400 070
- US-A- 4 894 012
- US-A- 5 527 182

## Beschreibung

Die Erfindung betrifft ein Verfahren nach dem Oberbegriff des Anspruchs **1,** eine Vorrichtung nach dem Oberbegriff des Anspruchs **16** und eine Verwendung dieser Vorrichtung nach dem Anspruch **28.**

Dokument WO 96 10371 wird als nächsteliegender Stand der Technik betrachtet. Dokument WO 96 10371 offenbart ein Verfahren zur Ermittlung der Form eines mit einem Zahnersatzteil zu versehenden Duplikats eines Restzahnbereichs, wobei die gegenseitigen Lagen der Duplikatsabschnitten und ihre Orientierungen ermittelt und die dabei ermittelten Daten gespeichert werden, wobei das Duplikat in Duplikatsabschnitte zertrennt wird und die Form der Duplikatsabschnitte einzeln ermittelt werden und die dabei ermittelten Daten gespeichert werden und wobei die Daten des Duplikats und die Daten der Duplikatsabschnitte zusammengeführt werden. Dokument WO 96 10371 offenbart eine Vorrichtung zur Ermittlung der Form eines mit einem Zahnersatzteil zu versehenden Duplikats eines Restzahnbereichs, mit einer ersten Einspannvorrichtung für das Duplikat, mit einer ersten berührungsfrei wirkenden Sensoreinrichtung, die zum Ermittel von den gegenseitigen Lagen der Zähnen und ihren Orientierungen ist, wobei die erste Sensoreinrichtung und die erste Einspannvorrichtung relativ zu einander bewegbar sind, mit einer zweiten Einspannvorrichtung für einzelne Abschnitte des Duplikats, mit einer zweiten Sensoreinrichtung, die zum Ermittel der Formen von Abschnitten des Duplikats geeignet ist, wobei die zweite Einspannvorrichtung relativ zur zweiten Sensoreinrichtung bewegbar ist, und mit einer Speichereinheit zum Speichern der von den beiden Sensoreinrichtungen ermittelten Daten.

Zahnersatzteile wurden während langer Zeit je nach ihrer Verwendung als Inlays, Onlays oder Brücken und je nach den im Einzelfall vertretbaren Kosten aus Amalgam, Gold oder Porzellan hergestellt. Bei Verwendung von Amalgam erfolgt die Formgebung in situ, das heisst im Mund des Patienten, indem die formbare Amalgammasse im unausgehärteten Zustand in die zu füllenden Kavitäten gebracht wird; auch ein gewisser Aufbau von Material ausserhalb von Kavitäten ist mit Amalgam mögtich, wobei die Formgebung entweder in der Art einer Modellierung am formbaren Amalgam oder mit Schleifmitteln am ausgehärteten Amalgam stattfindet. Bei Verwendung von Gold oder Porzellan erfolgt die Formgebung durch den Zahntechniker, im allgemeinen nach einem vorher im Mund des Patienten hergestellten Modelles bzw. Duplikates des herzustellenden Zahnersatzteiles.

Da sowohl Amalgam wie auch Gold oder Porzellan nicht in jeder Beziehung befriedigende Ergebnisse zeitigen, wird seit einiger Zeit versucht, Zahnersatzteile aus Kunststoff herzustellen. Dabei hat sich herausgestellt, dass die Haltbarkeit solcher Zahnersatzteile wesentlich verbessert werden kann, wenn der dazu verwendete Kunststoff mit Glasfasern verstärkt wird. Allerdings werden bis heute in der Zahnmedizin glasfaserverstärkte Kunststoffe nur wenig benutzt. Bekannte Zahnersatzteile werden mit Glasfaserbündeln hergestellt, die mit einem flüssigen, als Klebstoff wirkenden Kunststoff getränkt waren. Die Formgebung erfolgt manuell in der Art einer Modellierarbeit. Die physikalischen Eigenschaften solcher Zahnersatzteile sind infolge der manuellen Bearbeitungsschritte nicht gesichert, ausserdem kommt es bei den bekannten Materialien auch vor, dass während des Aushärtens Volumenveränderungen stattfinden.

In neuester Zeit werden Zahnersatzteile aus einem Material hergestellt, mit weichem eine automatisierte Fertigung möglich ist, und aus welchem Zahnersatzteile gefertigt werden können, die form- und volumenkonstant sind, und die Materialeigenschaften aufweisen, welche sich weder während der Bearbeitung noch im Gebrauch oder durch Alterung ändern.

Das neue Material ist ein glasfaserverstärkter Kunststoff, aus welchem durch einen speziellen Spritzgussvorgang Körper hergestellt werden, die Rohlinge für die Zahnersatzteile bilden. Die Formgebung der Zahnersatzteile erfolgt dann durch spanabhebende Bearbeitung, entweder in der Art einer Kopierfräsung oder mittels elektronisch gesteuerter Werkzeuge. Durch die Herstellung in einem speziellen Gussverfahren wird erreicht, dass die verstärkenden Glasfasern in der Kunststoffmasse je nach Bedarf angeordnet werden können. Nach der Aushärtungszeit sind die endgültigen physikalischen Eigenschaften des Materials erreicht, und diese ändern sich weder durch mechanische oder chemische Beanspruchung, noch während einer Bearbeitung, noch durch Alterung.

Als besonders geeigneter Kunststoff hat sich partiell aromatisches Polyamid erwiesen, dessen mechanische Eigenschaften durch die verstärkenden Glasfasern erhöht wird. So können beispielsweise ein Zug-E-Modul von 22 GPa und eine Brinellhärte von 280 MPa erreicht werden. Die Bruchlast beträgt ohne Verblendung etwa 1'150 N und kann mit Verblendung über 1'500 N erreichen. Zugleich ist dieses Material leicht; seine spezifische Masse beträgt etwa 1,7 g pro Kubikzentimeter, so dass es etwa 2,6 mal leichter ist als Titan, das schon als sehr leicht angesehen wird. Ausserdem ist es thermisch schlecht leitend, so dass aufgrund von Wärmeleitung entstehende Beschwerden der Patienten praktisch ausgeschlossen sind. Auch in ästhetischer Hinsicht ist das Material mehr als befriedigend; es ist transluszent, also lichtdurchlässig, und hat eine dem Zahnschmelz ähnliche Farbe.

Je nach der Verwendung des Rohlings zur Herstellung von Inlays, Kronen oder Brücken unterliegt das Material in seiner Endkonfiguration unterschiedlichen Arten von Beanspruchungen. Bei allseitig gleichen Beanspruchungen wird vorzugsweise ein Material verwendet, das isotrop oder mindestens quasiisotrop ist, worunter verstanden werden soll, dass es sich wie ein isotropes Material verhält. Dies kommt dadurch zustande, dass die Glasfasern im Kunststoff in ungerichteter Anordnung eingegossen sind.

Sind jedoch die Beanspruchungen, denen der Zahnersatzteil unterliegt, in gewissen Richtungen überwiegend, so wird vorzugsweise ein Material verwendet, das sich entsprechend anisotrop verhält. Ein solches kann erzeugt werden, indem die Glasfasern in gerichteter Anordnung eingegossen werden.

Zur Herstellung von Zahnersatzteilen aus Rohlingen mittels eines spanabhebenden Verfahrens muss die Form bzw. Berandung des mit Zahnersatzmaterial zu versehenden Restzahnbereichs bekannt sein. Der Restzahnbereich ist häufig ein einzelner Restzahn, der durch ein Inlay oder eine Krone ergänzt werden soll; in gewissen Fällen, wenn zum Beispiel Brücken angefertigt werden sollen, umfasst der Restzahnbereich mehrere Restzähne. Zu diesem Zwecke erfolgt herkömmlicherweise eine automatisierte Ermittlung dieser Berandung, wozu sich verschiedene Verfahren eignen. Angewendet werden beispielsweise eigentliche Abtastverfahren sowie berührungsfreie, gewissermassen optische Verfahren mit Linien-, Streifen- oder Musterprojektion, Holographie-Interferometrie, Graustufen-Auswertung, Abstandsmessung mittels verschiedenen Sensoren, Profilmessung und Tomographie-Röntgenbilderauswertung.

Da die Ermittlung der Form von Restzahnbereichen in situ, das heisst im Mund des Patienten, für diesen unangenehm und für den Zahnarzt mühsam ist und häufig nicht mit der erforderlichen Genauigkeit erfolgen kann, wird im allgemeinen zuerst ein Duplikat hergestellt und dann die Form des Duplikates des Restzahnbereiches erfasst. Aber auch diesem Verfahren haften zwei wesentliche Nachteile an. Zum einen können Schattenzonen bzw. tote Winkel oder Hinterschnitte nicht erfasst werden, weil sie Abtastinstrumenten nicht zugänglich sind, bzw. weil bei berührungsfreien Verfahren die direkte Verbindungslinie von Strahlungsquelle bzw. Sensor einerseits und abzutastendem Bereich anderseits unterbrochen ist, und zwar im allgemeinen durch eine andere Partie des abzutastenden Körpers selbst. Zum anderen ist in gewissen Fällen das Ergebnis der Abtastung nicht genügend genau, da es je nach den Abmessungen des abzutastenden Objektes stark variiert; die Auflösung digitaler Messsensoren wird üblicherweise nicht in Längenmassen wie Millimetern oder Metern ausgedrückt sondern in Bildpartikeln, die als Pixel bezeichnet werden. Als tatsächliche Auflösung ist der Quotient aus Messfeld-Grösse und Pixel-Zahl definiert. Ein Sensor mit 1000 Pixel wird somit eine 10 mm lange Strecke mit einer Auflösung von 10 µm Ermitteln, eine Strecke von 100 mm, die möglicherweise aus einer Vielzahl von Teilstrecken besteht, jedoch nur mit einer Auflösung von 0,1 mm, was einem Zehntel der zuerst genannten Auflösung entspricht, wobei diese geringere Auflösung den Anforderung der Zahnmedizin nicht entspricht.

Es ist somit **Aufgabe** der Erfindung, ein Verfahren, eine Vorrichtung zur Durchführung dieses Verfahrens und eine Verwendung der Vorrichtung aufzuzeigen, welche die Ermittlung der Form von Duplikaten von mit Zahnersatzteilen zu versehenden Restzahnberelchen unter Vermeidung der oben beschriebenen Nachteile ermöglicht wird.

Die **Lösung** dieser Aufgabe erfolgt durch die Merkmale der kennzeichnenden Teile der Patentansprüche **1** bzw. **16** bzw. **27** gelöst. Vorteilhafte Weiterbildungen des erfindungsgemässen Verfahrens und der erfindungsgemässen Vorrichtung sind durch die abhängigen Patentansprüche **6** bis **15** bzw. **17** bis **26,** eine Verwendung durch Anspruch **28** definiert.

Die Erfindung gemäss den soeben erwähnten Patentansprüchen beruht auf folgenden Überlegungen: die Auflösung, mit der die einzelnen Bereiche der abzutastenden Berandungen erfasst werden, muss sehr hoch sein, während die relative Position dieser Bereiche nicht unbedingt mit einer gleich hohen Auflösung gemessen werden muss. Bekannte Abformungsmethoden zur Herstellung eines Duplikates der abzutastenden Region genügen diesen Genauigkeitsanforderungen. Im Gegensatz zum Original, nämlich einem Bereich eines Gebisses, lässt sich ein Duplikat in mehrere Duplikatsabschnitte zerteilen, wonach die Form jedes einzelnen dieser Duplikatsabschnitte ermittelt wird. Bei geeigneter Abschnittseinteilung fallen die Probleme der Schattenzonen und Hinterschnitte weg. Beim erfindungsgemässen Verfahren werden nicht nur die Formen der Duplikatsabschnitte sondern auch, wie bei herkömmlichen Verfahren, die Form des Duplikates selbst ermittelt. Im einfachsten Fall ist es möglich, die entsprechenden Formen in einer einzigen relativen Anordnung zum Sensor mit genügender Genauigkeit zu ermitteln und anschliessend zu speichern.

Die bei der Ermittlung der Formen der einzelnen Duplikatsabschnitte ermittelten Duplikatsabschnittsdaten charakterisieren die einzelnen Partien des Restzahnbereichs in genauerer Weise als die Daten des gesamten Duplikates, hingegen lassen sich aus diesen Duplikatsabschnittsdaten die Relativlagen der einzelnen Duplikatsabschnitte nicht ermitteln; dies ist aber unerheblich, da die Genauigkeit der das gesamte Duplikat betreffenden Daten zur Bestimmung der Relativlagen der einzelnen Duplikatsabschnitte ausreicht.

Bei vorbekannten Verfahren erfolgt die Herstellung von Zahnersatzteilen gelegentlich durch direktes Kopieren in der Art von Kopierfräsen, was einer Analog-Verknüpfung entspricht. Dazu müssen die Daten, welche die Form des zu erzeugenden Zahnersatzteiles definieren, nicht notwendigerweise digitalisiert werden; auch wenn die zu ermittelnde Form des Restzahnbereiches sehr einfach ist oder wenn gewisse Ungenauigkeiten in Kauf genommen werden, und daher die Formermittlung nur in einer einzigen relativen Lage des Duplikates bzw. der Duplikatsabschnitte zum Sensor erfolgt, müssen die Daten, welche die ermittelten Formen charakterisieren, nicht unbedingt als digitale Werte gespeichert werden. Im allgemeinen ist es aber unumgänglich, zur Ermittlung der genauen Form des Restzahnbereiches die Duplikatsabschnitte und das gesamte Duplikat sukzessive in mehrere relative Anordnungen zum Sensor zu bringen, damit die Formen unter verschiedenen Winkeln vom Sensor erfasst werden können. Die ermittelten Daten müssen in diesem Fall als Digitaldaten gespeichert werden, damit anschliessend in einem sogenannten 3-dimensionalen Matching-Verfahren einerseits die unterschiedlichen Daten der einzelnen Duplikatsabschnitte und anderseits die unterschiedlichen Daten des Duplikates zusammengeführt, wodurch man ein präzises Abbild des Restzahnbereiches in digitaler Darstellung erhält. Die ermittelten Daten müssen deshalb digitalisiert werden, damit sie zur Bearbeitung durch eine elektronische Datenverarbeitungseinrichtung tauglich werden. Die elektronische Datenverarbeitungseinrichtung erlaubt eine Akkumulation bzw. Speicherung von Daten mehrerer konsekutiv durchgeführter Teil-Ermittlungen z.B. aus verschiedenen Winkeln, und anschliessend eine 3D-Zusammenführung der Daten des Gesamt-Duplikates mit den Daten der Duplikatsabschnitte, so dass sich die Ermittlung der Form des Restzahnbereiches insgesamt mit sehr stark erhöhter Genauigkeit durchführen lässt. Eine Digitalisierung der Daten ist auch in Fällen, in denen ein 3D-Matching nicht erforderlich ist, vorteilhaft, um die Daten in eine Form zu bringen, in welcher sie in einfacher Weise mittels einer EDV-Anlage bearbeitet werden können. Ausserdem ermöglicht die Digitalisierung der Daten, dass diese in einer Speichereinheit zur späteren Nutzung gespeichert werden können.

Wie schon erwähnt, sind, auch wenn mit gleicher Auflösung gearbeitet wird, die die Duplikatsabschnitte charakterisierenden Daten genauer als die das Duplikat charakterisierenden Daten. Eine hohe Genauigkeit für die letzteren wird ohnehin nicht benötigt. Es ist daher rationell, die das Duplikat charakterisierenden Daten in einem Verfahren mit geringerer Auflösung zu ermitteln als die die Duplikatsabschnitte charakterisierenden Daten.

Für das erfindungsgemässe Verfahren werden, wie erwähnt, einerseits das Duplikat und anderseits die Duplikatsabschnitte benötigt. Obwohl man zuerst die Form des Duplikates ermitteln und dieses anschliessend in Duplikatsabschnitte zerteilen kann, ist es doch rationeller, über zwei Duplikate zu verfügen, von denen eines zerteilt wird.

Beide Duplikate könnten primäre Duplikate sein, doch ist es für den Patienten angenehmer, wenn nur das erste Duplikat ein primäres Duplikat ist und das zweite Duplikat ein nach dem ersten Duplikat hergestelltes sekundäres Duplikat ist.

Im allgemeinen erfolgt die Zerteilung des Duplikates zahnweise.

Die Ermittlung der Formen der Duplikatsabschnitte wird vorzugsweise in der Reihenfolge durchgeführt, die die Duplikatsabschnitte im gesamten Duplikat einnehmen.

Um genaue Daten zu erhalten, erfolgt die Ermittlung der Form des Duplikates sowie jedes Dupükatsabschnittes vorzugsweise nicht nur In mehreren Ebenen sondern auch in mehreren relativen Winkellagen zum Sensor.

Die Vorrichtung zur Durchführung des erfindungsgemässen Verfahrens umfasst Mittel zum Einspannen des Duplikats und der Duplikatsabschnitte, also eine Duplikatseinspannvorrichtung und eine Einspannvorrichtung zur Aufnahme von mindestens einem, vorzugsweise aber mehreren Duplikatsabschnitten. Im weiteren umfasst die erfindungsgemässe Vorrichtung eine Sensoreinrichtung, welche sich sowohl zur Ermittlung der Formen des Duplikates wie auch der Duplikatsabschnitte eignet. Die Mittel zum Einspannen des Duplikats und der Duplikatsabschnitte einerseits und die Sensoreinrichtung anderseits sind relativ zueinander bewegbar. Schliesslich umfasst die Vorrichtung eine Speichereinheit zum Speichern der ermittelten Daten als digitale Grössen.

Als Sensoreinrichtung wird vorzugsweise eine berührungsfrei wirkende Sensoreinrichtung verwendet, vorzugsweise eine Lichtmusterquelle wie zum Beispiel eine Laserlinienquelle, welche mit einer digitalen Kamera gekoppelt ist.

Die Ermittlung der Daten kann in rationeller Weise erfolgen, wenn die Abschnitts-Einspannvorrichtung so ausgebildet ist, dass sie gleichzeitig mehrere Duplikatsabschnitte aufnehmen kann, und zwar vorzugsweise in derselben Reihenfolge und in annähernd derselben Lage, in der sie sich im Duplikat selbst befinden.

Zur Ermittlung genauer Daten ist es notwendig, dass sich die Sensoreinheit einerseits und die Duplikatsabschnitte sowie vorzugsweise auch das Duplikat anderseits relativ zueinander bewegen lassen. Es hat sich als günstig erwiesen, eine innerhalb der Vorrichtung ortsfeste Sensoreinrichtung und eine bewegliche die Duplikatseinspannvorrichtung sowie eine bewegliche Abschnittseinspannvorrichtung zu verwenden. Zu diesem Zweck werden die Duplikatseinspannvorrichtung und die Abschnittseinspannvorrichtung einzeln oder zusammen auf einem Tisch angeordnet, der über zwei orthogonale Führungen abgestützt ist, so dass er sich, motorisch angetrieben, längs dieser orthogonalen Richtungen verschieben kann. Dadurch lassen sich die Formen des Duplikates und der Duplikatsabschnitte in parallelen Ebenenscharen ermitteln.

Um die Formen des Duplikates und der Duplikatsabschnitte genauer und umfassender zu ermitteln, müssen diese für den Sensor in verschiedenen Winkellagen einsehbar sein. Damit dies möglich ist, werden die Duplikatseinspannvorrichtung und die Abschnittseinspannvorrichtungen so ausgebildet, dass sie, motorisch angetrieben, um eine Achse drehbar sind, welche im beispielsweise parallel zur Fläche des Tisches und meist, aber nicht zwingend, parallel zu einer der beiden orthogonalen Richtungen verläuft.

Zur Feststellung der Längslagen des Tischen und der Winkellagen der Duplikatseinspannvorrichtung und der Duplikatsabschnittsvorrichtung sind im allgemeinen entsprechende Positionsgeber, zum Beispiel Encoder-Einheiten, vorgesehen.

Besonders vorteilhafte Weiterbildungen des Verfahrens und der Vorrichtung nach der Erfindung werden zur Herstellung von Zahnersatzteilen benutzt.

Grundsätzlich umfasst die Herstellung eines Zahnersatzteiles - mit Ausnahme derjenigen Verfahren, bei denen nicht ein plastisch verformbares und später aushärtendes Material direkt in die zu füllende Kavität eines Zahnes oder auf einen Zahnrest gebracht wird, - zwei Teilvorgänge, nämlich
- erstens die oben beschriebenen Ermittlung der Form des mit dem Zahnersatzteil zu versehenden Restzahnbereiches, welche die relevanten Zonen der Form des Zahnersatzteiles entspricht, und
- zweitens die im Folgenden beschriebene Erzeugung des Zahnersatzteiles aufgrund der ermittelten Grössen.

Unter den relevanten Zonen werden jene Zonen verstanden, die bei der Montage Im Munde des Patienten an noch vorhandenem Restzahnmaterial zur Anlage kommen. Andere Zonen, die nicht an Restzahnbereichen zur Anlage kommen, sollten zwar wenn möglich auch präzis gefertigt werden, nämlich so, dass sie der ursprünglichen Zahnform entsprechen; sie lassen sich zwar nach ihrer Montage durch Schleifen korrigieren, aber dadurch erreichte Präzision ist geringer als bei den relevanten Zonen.

Die Ermittlung der Form des herzustellenden Zahnersatzteiles und die tatsächliche Formgebung des Zahnersatzteiles werden herkömmlicherweise verknüpft, indem die ermittelten Werte zur Steuerung der Bearbeitungswerkzeuge verwendet werden. Dies kann, wie schon erwähnt, durch eine Art Kopierfräsen erfolgen. Aus der **EP-0 376 873** Ist ferner bekannt, eine Fräsereinrichtung an eine Abtastvorrichtung, welche Ergebnisse in digitaler Form liefert, anzukoppeln. Diese Ergebnisse werden gespeichert und zur Steuerung der Fräsereinrichtung benutzt.

Mit den erwähnten besonders vorteilhaften Weiterbildungen der Erfindung ist beabsichtigt, die herkömmlichen Verfahren zur Herstellung von Zahnersatzteilen und die Vorrichtungen zur Durchführung dieser Verfahrens so zu verbessern, dass die Herstellung der Zahnersatzteile schneller, genauer und vor allem unabhängig von handwerklichen Geschick eines Zahnarztes oder Zahntechnikers durchgeführt werden kann.

Während die vorbekannte Abtastvorrichtung gemäss der **EP-0 376 873** manuell gesteuert wird und eine eigene Speichereinheit aufweist, und die bekannten Fräsereinrichtungen ebenfalls eine eigene Steuereinheit besitzen, erfolgt bei der bevorzugten Weiterbildung der Erfindung die Ermittlung der Form des mit dem Zahnersatzteil zu versehenden Restzahnbereichs, kurz Formermittlung genannt, und die eigentliche Erzeugung des Zahnersatzteiles, indem man die Formermittlungsvorrichtung und eine Fräsereinrichtung über eine EDV-Anlage koppelt. Man integriert somit die Formermittlung, die eigentliche Fertigung des Zahnersatzteiles und die Steuerung und Überwachung des gesamten Ablaufes.

Bei der besonders bevorzugten Weiterbildung der Erfindung erfolgt die Herstellung des Zahnersatzteiles auf Grund der Formermittlung nach der Erfindung. Die Formermittlung des Restzahnbereiches könnte aber grundsätzlich in jeder Weise durchgeführt werden könnte, welche die entsprechenden Ergebnisse als Digitaldaten liefert.

Vorzugsweise erfolgt die Verbindung der Verfahrensschritte der Formermittlung und der eigentlichen Fertigung des Zahnersatzteiles in einer Weise, die es erlaubt, vor dem Verfahren gewisse verfahrensrelevante Angaben festzulegen und während des Verfahrensablaufs in das Verfahren einzugreifen, um zusätzliche Angaben vorzunehmen oder frühere Angaben zu verändern.

Die Durchführung des Verfahrens wird bedeutend erleichtert, wenn der gesamte Verfahrensablauf einschliesslich der Verfahrensvorbereitung und des Verfahrensergebnisses visualisierbar ist.

Zur Durchführung des Verfahrens wird vorzugsweise eine verfahrensspezifische Software benutzt.

Die bevorzugte Weiterbildung der erfindungsgemässen Vorrichtung, mit welcher die Herstellung von Zahnersatzteilen erfolgt, umfasst eine Formermittlungsvorrichtung und eine Fräsereinrichtung bzw. eine andere geeignete Fertigungseinrichtung zur eigentlichen Fertigung des Zahnersatzteiles, sowie eine EDV-Einrichtung. Die EDV-Einrichtung koppelt die Formermittlungsvorrichtung mit der Fräsereinrichtung und umfasst auch die Speichereinheit für die Ergebnisse der Formermittlungsvorrichtung und die Steuereinheit zum Steuern der Fertigungseinrichtung. Die EDV-Anlage übernimmt somit nicht nur die Verbindung von Sensoreinrichtung und Fräsereinrichtung, sondern auch die Funktion der Steuereinheit der Formermittlungsvorrichtung sowie die Funktion der Steuereinheit der Fräsereinrichtung. In dieser neuen Kombination mit den drei seriellen Bausteinen Formermittlungsvorrichtung, EDV-Anlage und Fräsereinrichtung erfolgt also eine Konzentration aller Steuer- und Überwachungsvorgänge in der EDV-Anlage, so dass die Formermittlungsvorrichtung und die Fräsereinrichtung keine eigentlichen EDV-Einheiten besitzen müssen. Dies ist sowohl für die Herstellung wie auch für den Unterhalt der gesamten Anlage vorteilhaft und erlaubt es, die gesamte Herstellung des Zahnersatzteiles zentral zu steuern und zu überwachen.

Vorzugsweise umfasst die EDV-Einrichtung der neuen Anlage eine Eingabeeinheit, im allgemeinen in Form einer Tastatur mit einer Maus oder einem Trackball. Die Eingabeeinheit dient insbesondere der Eingabe von Angaben vor und während der Formermittlung und der eigentlichen Fertigung des Zahnersatzteiles und erlaubt es auch, in ein laufendes Verfahren einzugreifen bzw. frühere Angaben zu korrigieren, die gleich oder in anderer Weise, beispielsweise ab Disketten oder CDs, gemacht wurden.

Die Bedienung der Anlage wird ausserordentlich erleichtert, wenn die EDV-Einrichtung eine Monitoreinheit enthält, mittels welcher das gesamte Verfahren der Formermittlung und der eigentlichen Fertigung visualisiert werden kann.

Die zur Herstellung des Zahnersatzteiles notwendigen Anweisungen können einzeln via Eingabeeinheit der EDV-Einrichtung für jeden Zahnersatzteil oder für Gruppen von Zahnersatzteilen eingegeben werden. Für eine rationelle Verwendung der Anlage empfiehlt es sich aber, die EDV-Einrichtung mit einer geeigneten Software auszustatten.

Die Software sollte das gesamte Wissen aller Fachleute enthalten, die herkömmlicherweise mit der Herstellung eines Zahnersatzteiles befasst sind, insbesondere das Wissen des Dentisten, der die Art des herzustellenden Zahnersatzteiles bestimmt und die Form des entsprechenden Restzahnbereiches ermittelt, des Zahntechnikers, des Werkstoffspezialisten, der das Material für den Zahnersatzteil herstellt bzw. auswählt, und des Maschinisten, dem die eigentliche Fertigung des Zahnersatzteils obliegt. Dank der neuen Software können alle diese und ggfs. weitere Vorgänge durch eine Person ohne spezifisches Fachwissen durchgeführt werden.

Die bevorzugte Weiterbildung der Vorrichtung, mit welcher Zahnersatzteile hergestellt werden, kann verwendet werden zur Herstellung von Zahnersatzteilen aus den verschiedensten Materialien wie beispielsweise Metall, Keramik oder Kunststoff, beispielsweise ein mit Glasfasern verstärkter Kunststoff

Weitere Einzelheiten und Vorteile der Erfindung werden im folgenden anhand der Beschreibung und mit Bezug auf die Zeichnung ausführlich beschrieben. Es zeigt:
- **Fig. 1**: einen Rohling aus einem für Zahnersaatzteile geeigneten Material, in einem Schaubild;
- **Fig. 2**: den in Fig. 1 dargestellten Rohling in einem Schnitt;
- **Fig. 3**: ein Duplikat eines mit einem Zahnersatzteil zu versehenden Restzahnbereich und eine Sensoreinrichtung, in vereinfachter schematischer Darstellung;
- **Fig. 4**: ein primäres und ein sekundäres, teilweise in Abschnitte zerteiltes Duplikat eines Restzahnbereiches, in vereinfachter, schematischer Darstellung;
- **Fig. 5**: eine Sensoreinrichtung bei der Ermittlung der Form eines in einer Abschnitts-Einspannvorrichtung eingespannte Duplikatsabschnittes, in vereinfachter, schematischer Darstellung;
- **Fig. 6A - 6D**: das Formermittlungsraster für die Duplikatsabschnitte, in einer Winkellage des Duplikatsabschnittes, in vereinfachter, schematischer Darstellung;
- **Fig. 7**: die Sensoreinrichtung bei der Ermittlung der Form eines Duplikates, in vereinfachter, schematischer Darstellung;
- **Fig. 8**: das Formermittlungsraster für das Duplikat, in vereinfachter, schematischer Darstellung;
- **Fig. 9A-9C**: das 3D-Matching in vereinfachter, schematischer Darstellung;
- **Fig. 10**: eine Vorrichtung zur Ermittlung der Form eines mit einem Zahnersatzteil zu versehenden Restzahnbereiches;
- **Fig. 11A**: eine Vorrichtung zur Herstellung eines Zahnersatzteils nach dem Stand der Technik, in schematischer Darstellung;
- **Fig. 11B**: eine Vorrichtung zur Herstellung eines Zahnersatzteiles nach der Erfindung, in gleicher Darstellung wie Fig. 11A;
- **Fig. 12**: der Ablauf der Herstellung von Zahnersatzteilen in der Vorrichtung nach der Erfindung, in schematischer Darstellung, und
- **Fig. 13A-13D**: Beispiele des visualisierten Ablaufs der Herstellung von Zahnersatzteilen.

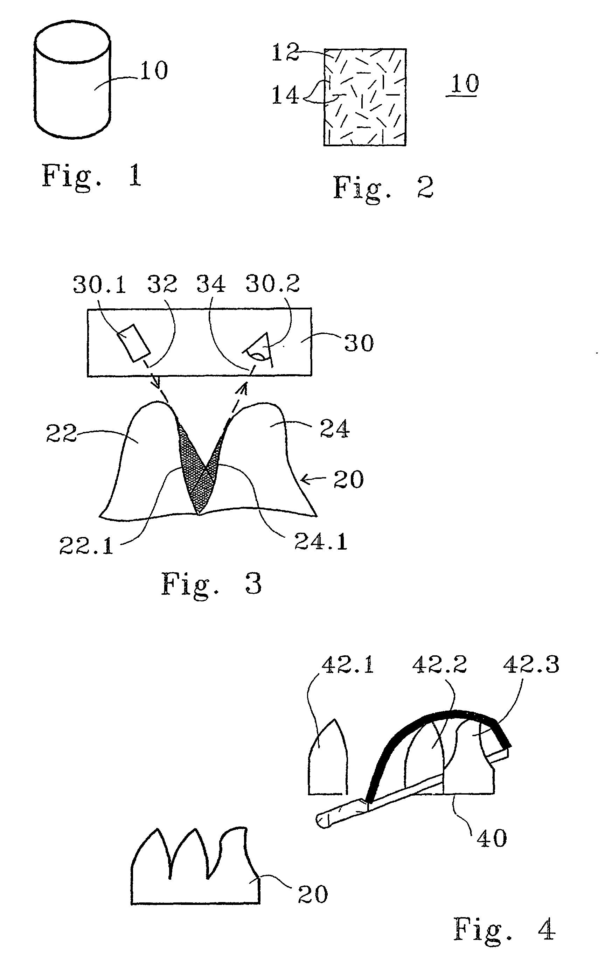

**Fig. 1** zeigt einen Rohling **10** aus einem geeigneten Material, aus welchem sich mittels spanabhebender Bearbeitung ein Zahnersatzteil fertigen lässt. Der Rohling **10** kann, wie in **Fig. 1** dargestellt, in der etwaigen Grösse eines Zahnersatzteiles, mit entsprechenden Bearbeitungszuschlägen, oder in grösseren Abmessungen für mehrere Zahnersatzteile als Stange oder Block gegossen werden. Rohlinge in der ungefähren Grösse Zahnersatzteilen können direkt weiterverarbeitet werden.

Der Rohling **10** besteht aus einem geeigneten Kunststoff **12,** der durch Abschnitte eingegossener Glasfasern **14** verstärkt ist. Infolge der Herstellung durch ein spezielles Giessverfahren befinden sich die Glasfasern **14** im Kunststoff **12** gemäss **Fig. 2** je nach den Anforderungen an einzelne Zahnersatzteile in gerichteter oder ungerichteter Anordnung, so dass das Material als anisotrop bzw. isotrop bezeichnet werden kann und sich unter Beanspruchungen auch entsprechend verhält.

**Fig. 3** erläutert das Problem der Ermittlung der Form eines Duplikates **20** eines Restzahnbereiches bei Vorhandensein von Schattenzonen und Hinterschneidungen. Das dargestellte Duplikat **20** umfasst einen Restzahnbereich mit zwei Zähnen **22** und **24.** Die Bereiche **22.1** und **24.1** der Oberflächen der Zähne **22, 24,** die an den schraffierten Bereich angrenzen, lassen sich bei der Ermittlung der Form durch eine Sensoreinrichtung **30** mit einer Lichtquelle **30.1** und einem Sensorteil **30.2** nicht erfassen, weil die Linien **32** und **34** durch die Zähne **22** bzw. **24** unterbrochen werden.

In **Fig. 4** ist das Duplikat **20** in einer anderen Ausführung als in **Fig. 3,** nämlich mit drei Zähnen, dargestellt. Beim diesem Duplikat **20** handelt es sich um ein primäres Duplikat. Im weiteren ist in **Fig. 4** ein sekundäres Duplikat **40** dargestellt, welches mittels einer als Säge dargestellten Trenneinrichtung in Duplikatsabschnitte **42,** im vorliegenden Fall in die Duplikatsabschnitte **42.1, 42.2** und **42.3** zerteilt wird.

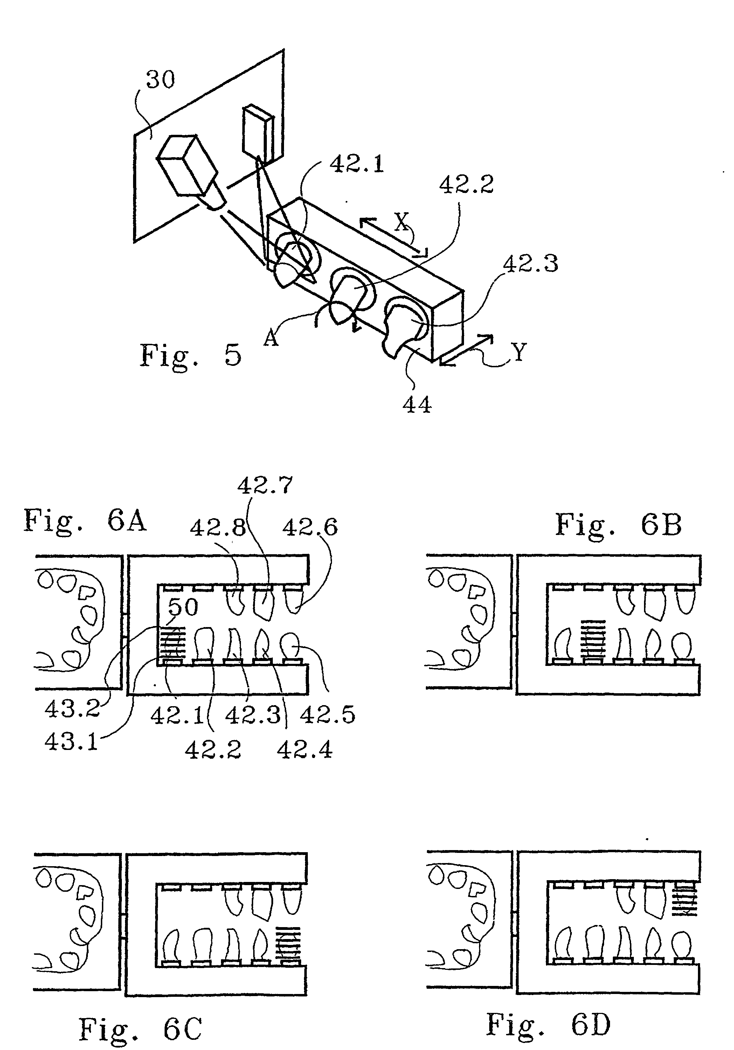

Gemäss **Fig. 5** werden die Duplikatsabschnitte **42.1, 42.2, 42.3** in gegenseitigen Abständen einzeln in Einheiten einer Abschnitts-Einspannvorrichtung **44** im Wahrnehmungsbereich der Sensoreinrichtung **30** eingespannt. Die Abschnitts-Einspannvorrichtung **44** lässt sich in Richtung der Doppelpfeile **X** und **Y** verschieben und die Duplikatsabschnitte **42.1, 42.2, 42.3** lassen sich in den Einheiten der Abschnitts-Einspannvorrichtung **44** entsprechend dem Pfeil **A** beidsinnig drehen. Die Duplikatsabschnitte **42.1, 42.2, 42.3** befinden sich in der Abschnitts-Einspannvorrichtung **44** in gleicher Reihenfolge wie im Duplikat **40,** jedoch nicht in gleicher gegenseitiger Lage. Da die Abstände zwischen den Duplikatsabschnitten **42.1, 42.2, 42.3** grösser sind als im unzerteilten Duplikat **40,** und da sich die Abschnitts-Einspannvorrichtung **44** in der soeben beschriebenen Weise relativ zur Sensoreinrichtung **30** bewegen lässt, gibt es bei der Ermittlung der Form der Duplikatsabschnitte **42.1, 42.2, 42.3** keine nicht einsehbaren Zonen entsprechend den Bereichen **22.1** und **24.1** gemäss **Fig. 3.**

Die **Fig. 6A** bis **6D** zeigen das Raster der Ermittlung der Formen der einzelnen Duplikats-Abschnitte **42.1** bis **42.8,** die in der Abschnitts-Einspannvorrichtung **44** angeordnet sind. Der Duplikatsabschnitt **42.1** wird gemäss **Fig. 6A** optisch und berührungsfrei vom Grund **43.1** zum freien Ende **43.2** in mehreren parallelen Ebenen **50** mit gegenseitigen Abständen von etwa 1 bis 30 µm abgetastet, und zwar erfolgt eine solche Abtastung in mehreren verschiedenen Winkellagen der Duplikatsabschnitte. **42.1** bis **42.8.** Nach der Abtastung des Duplikatsabschnitts **42.1** wird die Duplikats-Einspannvorrichtung **44** verschoben und es werden zuerst der Duplikatsabschnitt **42.2** und anschliessend, nach jeweiliger weiterer Verschiebung der Abschnitts-Einspannvorrichtung **44,** die weiteren Duplikatsabschnitte **42.3 bis 42.8** in gleicher Weise abgetastet. In den **Fig. 6B, 6C** und **6D** sind die Abtastvorgänge der Duplikatsabschnitte **42.2, 42.5** und **42.8** dargestellt.

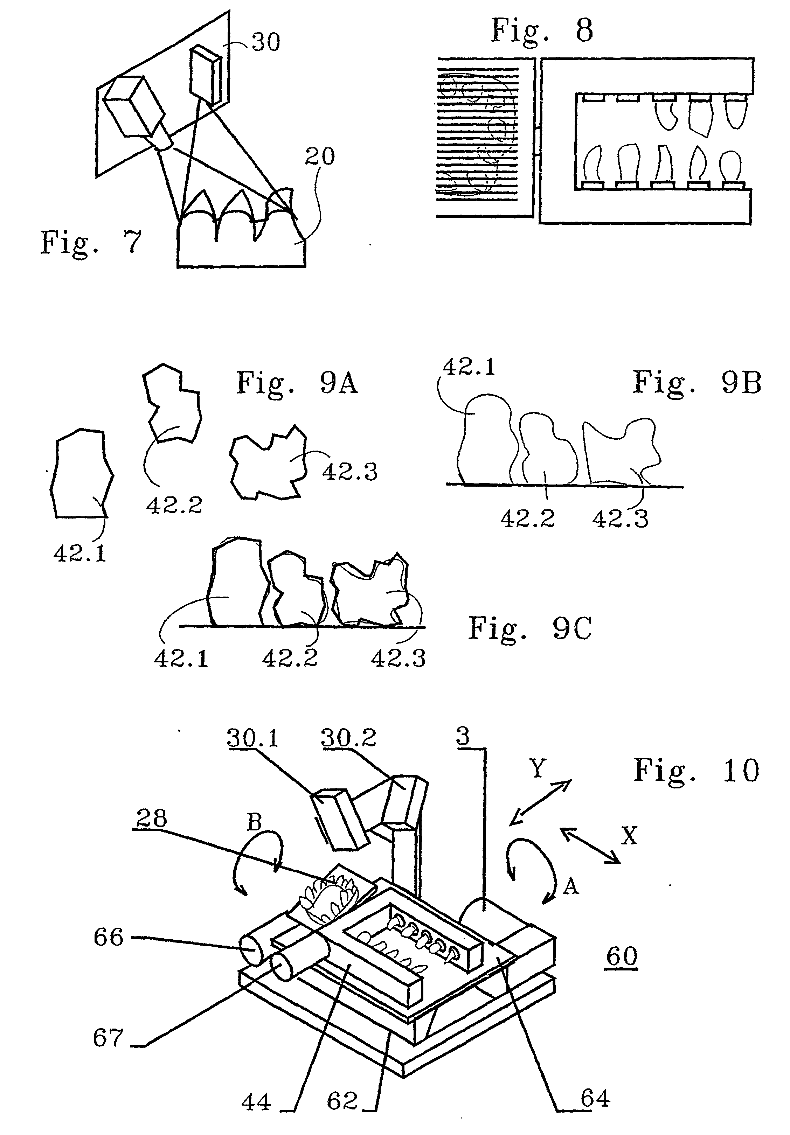

**Fig. 7** zeigt das Duplikat **20** während seiner Abtastung durch die Sensoreinrichtung **30.** Wie die Duplikatsabschnitte **42** wird gemäss **Fig. 8** auch das Duplikat in mehreren Ebenen und unter mehreren Winkeln abgetastet.

Die Abtastung der einzelnen Duplikatsabschnitte **42** erfolgt mit hoher Auflösung und liefert als Ergebnis gemäss **Fig. 9A** ein verhältnismässig genaues Abbild der Form der Duplikatsabschnitte **42.** Die Abtastung des Duplikats **20** selbst erfolgt mit einer Auflösung, die weit geringer ist als die Auflösung bei der Abtastung der Duplikatsabschnitte **42** und liefert als Ergebnis gemäss **Fig. 9B** ein viel ungenaueres Abbild des Duplikates **20,** das aber ausreicht, um Ort und Lage der Duplikatsabschnitte **42** im Duplikat **20** auszumachen. In einem 3D-Matching werden sodann die präzisen Ergebnisse der Abtastung der Duplikatsabschnitte **42** und die weniger präzisen Ergebnisse der Abtastung des Duplikats **20** zusammengeführt, wie dies in den **Fig. 9A** bis **9C** schematisch dargestellt ist. **Fig. 9A** zeigt das Ergebnis der Abtastung der Duplikatsabschnitte **42,** umfassend drei Duplikatsabschnitte **42.1, 42.2, 42.3,** in gleicher Reihenfolge wie im Duplikat **20** jedoch in anderer gegenseitiger Lage als im Duplikat **20,** und zwar mit Einzelheiten der Formen. **Fig. 9B** zeigt das Ergebnis der Abtastung des Duplikats **20,** in welcher die gegenseitige Lage der drei Duplikatsabschnitte **42.1, 42.2, 42.3** erkennbar ist, während die Einzelheiten der Formen fehlen. **Fig. 9C** schliesslich zeigt das Ergebnis des 3D-Matching.

In **Fig. 10** ist eine Vorrichtung **60** zum Ermitteln der Form des Duplikates **20** dargestellt. Diese umfasst die Sensoreinrichtung **30.1, 30.2,** die Duplikats-Einspannvorrichtung **28** und die Abschnittseinspannvorrichtung **44.** Die beiden Einspannvorrichtungen **28** und **44** sind auf einem Tisch **64** angeordnet, welcher über orthogonale Gleitführungen **62** abgestützt ist und sich in Richtung der Doppelpfeile **X** und **Y** verschieben lässt. Im weiteren sind Motoren **66** und **3** vorgesehen, mittels welchen sich der Tisch **64** längs der parallelen Führungen **62** bewegen lässt. Ein weiterer Motor **67** ermöglicht die Rotation der Duplikatsabschnitte **42.1** bis **42.8** in Richtung des Doppelpfeiles **A** und die Rotation der Duplikats-Einspannvorrichtung **28** in Richtung des Doppelpfeiles **B.**

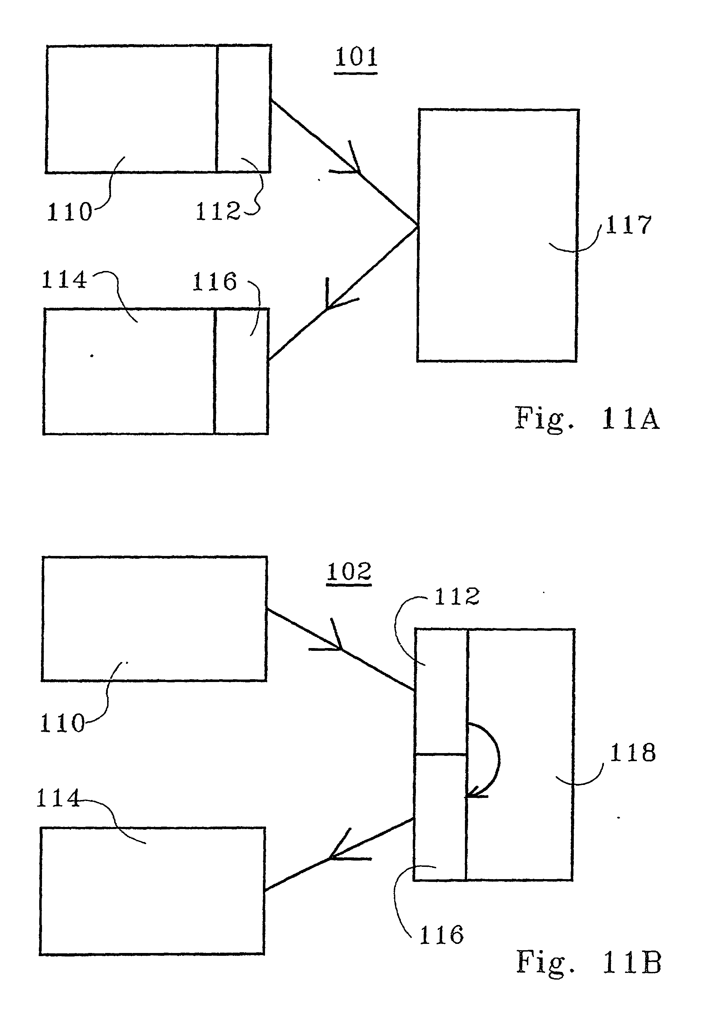

**Fig. 11A** zeigt schematisch eine herkömmliche Vorrichtung **101** zur Herstellung eines Zahnersatzteiles, welche aus drei herkömmlichen Bausteinen zusammengestellt ist, nämlich erstens aus einer Formermittlungsvorrichtung **110** mit einer Speichereinheit **112** zum Ermitteln der Form eines Duplikates eines mit dem Zahnersatzteil zu versehenden Restzahnbereiches und zum Speichern der Ergebnisse der Formermittlung, zweitens aus einer EDV-Einrichtung **117** und drittens aus einer Fertigungsmaschine **114**, im allgemeinen eine Fräsereinrichtung und/oder eine Schleifeinrichtung umfassend, mit einer Steuereinheit **116,** welche Fertigungsmaschine **114** zur eigentlichen Fertigung des Zahnersatzteils dient.

**Fig. 11B** zeigt eine entsprechende Vorrichtung **102;** hierbei handelt es sich um eine bevorzugte Weiterbildung der erfindungsgemässen Vorrichtung. Auch diese Vorrichtung **102** Anlage umfasst eine Formermittlungsvorrichtung **110** zum Ermitteln der Form eines Duplikates eines mit dem Zahnersatzteil zu versehenden Restzahnbereiches, eine Speichereinheit **112** zum Speichern der Ergebnisse der Formermittlung, eine EDV-Einrichtung **118** und eine Fertigungsmaschine **114** zur eigentlichen Fertigung des Zahnersatzteils und eine Steuereinheit **116** für die Fertigungsmaschine **114.**

Bei der herkömmlichen Vorrichtung **101** gemäss **Fig. 11A** gehören die Speichereinheit **112** zur Formermittlungsvorrichtung **110** und die Steuereinheit **116** zur Fertigungsmaschine **114;** die Formermittlungsvorrichtung **110** und die Fertigungsmaschine **114** sind durch die EDV-Einrichtung **117** gekoppelt; bei einer solchen Anordnung muss der Bediener im allgemeinen mehrere Eingabeeinheiten und ggfs. mehrere Monitore im Auge haben. Bei der erfindungsgemässen Vorrichtung **102** gemäss **Fig. 11B** sind die Formermittlungsvorrichtung **110** und die Fertigungsmaschine **114** durch die EDV-Einrichtung **118** gekoppelt, welche auch die Spelchereinheit **112** und die Steuereinheit **116** umfasst; bei der neuen Anlage hat daher der Bediener die Möglichkeit, sich auf eine Eingabeeinheit und einen Monitor zu konzentrieren..

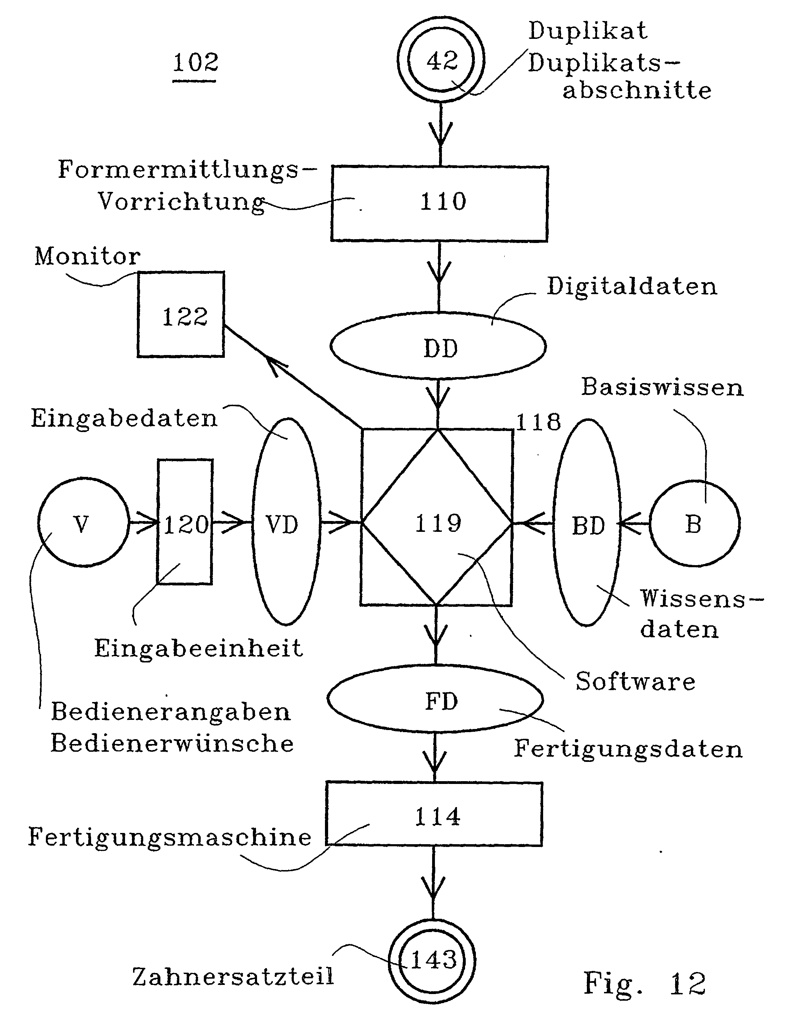

In **Fig. 12** ist die Wirkungsweise der neuen Vorrichtung **102** schematisch dargestellt. Die Formermittlungsvorrichtung **110** dient zur Ermittlung der Form des Duplikates bzw. der Duplikatsabschnitte **42** des Restzahnbereiches, der mit einem Zahnersatzteil **143** zu versehen ist. Die Ergebnisse, welche die Formermittlungsvorrichtung **110** liefert, werden als Digitaldaten **DD** gespeichert. Die eigentliche Fertigung des Zahnersatzteils **143** findet in der Fräsereinrichtung **114** statt. Die Steuerung der Formermittlungsvorrichtung **110** und der Fräsereinrichtung **114** sowie das weiter oben beschriebene 3D-Matching und die Speicherung der Ergebnisse der Formermittlung erfolgen mittels der EDV-Einrichtung **118,** welche eine spezifische Software **119** enthält. Die EDV-Einrichtung **118** ist durch eine Eingabeeinrichtung **120** und eine Monitoreinrichtung **122** ergänzt. Die Software **119** enthält das fachübergreifende Basiswissen **B,** welches zur Herstellung des Zahnersatzteiles **143** notwendig ist, die in Form von Basisdaten **BD** eingegeben wurde. Wünsche und Angaben **V** des Bedieners bzw. Verwenders der Anlage **102** werden mittels der Eingabeeinheit **120** in Form von Verwenderdaten **VD** eingegeben. Aus den Digitaldaten **DD,** den Basisdaten **BD** und den Verwenderdaten **VD** ermittelt die EDV-Einrichtung **118** mit Hilfe der Software **119** Fertigungsdaten **FD,** mit welchen der gesamte Vorgang der eigentlichen Fertigung des Zahnersatzteiles **143** in der Fertigungsmaschine bzw. Fertigungsmaschine **114** gesteuert wird.

Einzelheiten betreffend die Software **119** sind in den **Fig. 13A bis 13D** dargestellt. Die Software **119** umfasst unter anderem gewisse nicht im Zusammenhang mit einzelnen Zahnersatzteilen stehende grundlegende Daten, insbesondere Daten betreffend zur Verfügung stehende Materialien für Zahnersatzteile und Eigenschaften dieser Materialien, Daten betreffend durch die Fertigungsmaschine durchführbare Fertigungsvorgänge, die dabei verwendbaren Werkzeuge und die Art und Weise von deren Einsatz, wozu Daten über Zustellwinkel, Rotations- und Vorschubgeschwindigkeiten und tolerierbare Abnützung gehören. Die Software greift automatisch auf diese Daten zurück, ohne dass der Bediener bzw. Verwender der Anlage etwas davon verstehen muss oder bemerkt. Ferner führt die Software den Bediener bei der Ermittlung der Formen des Zahnersatzteiles, berechnet das 3D-Matching und dient generell als Zwischen- und Endspeichereinheit für die Formermittlungseinrichtung. Die Software führt auch bei der Eingabe von Wünschen aller Art, mit welchen im allgemeinen eine Auswahl zwischen verschiedenen Optionen getroffen wird oder eine vorbestimmte Auswahl vor oder während des gesamten Verfahrens abgeändert werden kann. Im weiteren dient die Software zur Festlegung der Fertigungsdaten zuhanden der Fertigungsmaschine. Schliesslich dient die Software auch dazu, Vorgänge im Bereich der Vorbereitung und Beendigung des Verfahrens sowie im Sinne der Archivierung einzelner Verfahren durchzuführen.

**Fig. 13A** zeigt das Bild, das zu Beginn der Herstellung des Zahnersatzteiles auf dem Monitor erscheint, und das im wesentlichen so aussieht wie eine Patientenkarte einer Kartei. Diejenigen Angaben, die sonst schriftlich bzw. zeichnerisch auf der Patientenkarte festgehalten werden, werden nun über die Tastatur eingegeben, von der Software ausgewertet und im späteren Verlauf des Verfahrens benützt. Die anschliessend durchzuführenden Schritte werden dann selbsttätig aufgerufen und dadurch der Bediener geführt. Gemäss Fig. **13A** ist der herzustellende Zahnersatzteil eine Brücke.

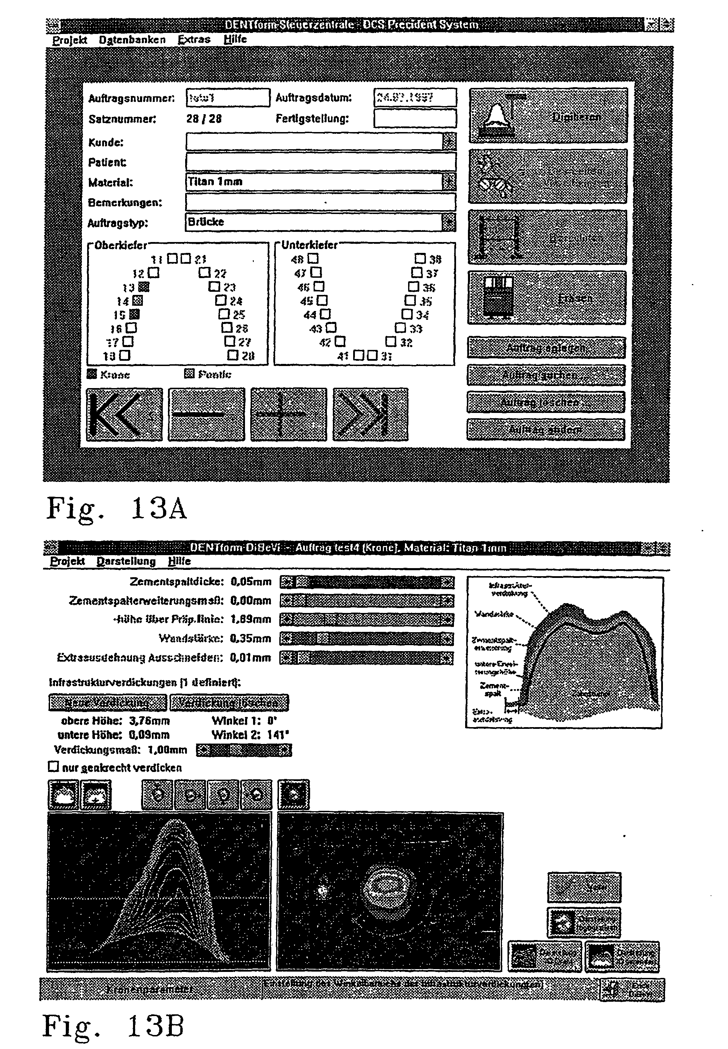

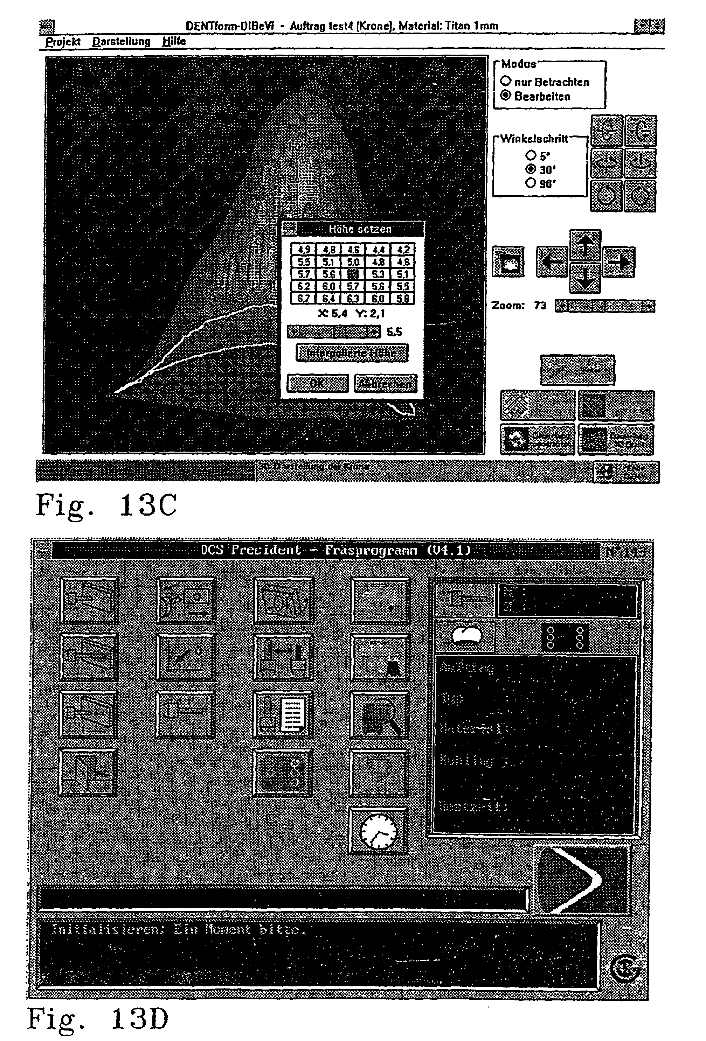

Als Beispiel zeigen die **Fig. 13B, 13C** und **13D** die Vorgänge bei der Herstellung einer Krone.

Die mit Bezug auf die **Fig. 1 bis 13** erläuterten Verfahrensschritte, Vorrichtungen und Vorrichtungsbestandteile sind ausdrücklich nur als Beispiele angeführt. Der Rahmen der Erfindung umfasst aber zu allen Einzelheiten zahlreiche weitere Varianten.

## Patentansprüche

1. Verfahren zur Ermittlung der Form eines mit einem Zahnersatzteil **(143)** zu versehenden Duplikats **(20)** eines Restzahnbereichs, wobei die Form des Duplikats **(20)** ermittelt und die dabei ermittelten Daten gespeichert werden,
**dadurch gekennzeichnet,**
- **dass** das Duplikat **(20)** in Duplikatsabschnitte **(42.1** bis **42.8)** zertrennt wird;
- **dass** die Form der Duplikatsabschnitte **(42.1** bis **42.8)** einzeln ermittelt werden und die dabei ermittelten Daten gespeichert werden; und
- **dass** die Daten des Duplikats **(20)** und die Daten der Duplikatsabschnitte **(42.1** bis **42.8)** zusammengeführt werden.

2. Verfahren nach Patentanspruch **1,**
**dadurch gekennzeichnet,**
**dass** die Form der Duplikatsabschnitte **(42.1** bis **42.8)** unter verschiedenen Winkeln erfasst wird und die ermittelten Daten als Digitaldaten **(DD)** gespeichert und mittels eines 3D-Matching-Verfahrens zusammengeführt werden.

3. Verfahren nach Patentanspruch **1,**
**dadurch gekennzeichnet,**
**dass** die Form des Duplikats **(20)** unter verschiedenen Winkeln erfasst wird und die ermittelten Daten als Digitaldaten **(DD)** gespeichert und mittels eines 3D-Matching-Verfahrens zusammengeführt werden.

4. Verfahren nach Patentanspruch **2** und **3,**
**dadurch gekennzeichnet,**
die ermittelten Daten der Duplikatsabschnitte **(42.1** bis **42.8)** und des Duplikats **(20)** zusammengeführt werden.

5. Verfahren nach den Patentansprüchen **2** und **3,**
**dadurch gekennzeichnet,**
**dass** die Ermittlung der Form des Duplikates **(20)** mit geringerer Genauigkeit erfolgt als die Ermittlung der Form jedes Duplikatsabschnittes **(42.1** bis **42.8).**

6. Verfahren nach mindestens einem der Patentansprüche **1** bis **5,**
**dadurch gekennzeichnet,**
**dass** ein zweites Duplikat **(40)** hergestellt wird, wonach eines der beiden Duplikate **(20** oder **40)** in die Duplikatsabschnitte **(42.1** bis **42.8)** zerteilt wird.

7. Verfahren nach Patentanspruch **6,**
**dadurch gekennzeichnet,**
**dass** das zweite Duplikat **(40)** sekundär aufgrund des ersten Duplikates **(20)** hergestellt wird.

8. Verfahren nach mindestens einem der Patentansprüche **1** bis **7,**
**dadurch gekennzeichnet,**
**dass** die Zerteilung des Duplikats **(40)** in die Duplikatsabschnitte **(42.1** bis **42.8)** zahnweise bzw. zahngruppenweise erfolgt.

9. Verfahren nach mindestens einem der Patentansprüche **1** bis **8,**
**dadurch gekennzeichnet,**
**dass** die Duplikatsabschnitte **(42.1** bis **42.8)** in derjenigen Reihenfolge abgetastet werden, die sie im unzerteilten Duplikat **(40)** eingenommen haben.

10. Verfahren nach mindestens einem der Patentansprüche **1** bis **9,**
**dadurch gekennzeichnet,**
**dass** die Ermittlung der Form des Duplikates **(20)** und jedes der Duplikatsabschnitte **(42.1** bis **42.4)** unter mehreren Winkellagen und in jeder Winkellage in einer Vielzahl paralleler Ebenen erfolgt.

11. Verfahren nach mindestens einem der Patentansprüche **1** bis **10,**
**dadurch gekennzeichnet,**
- **dass** zur Herstellung des Duplikats die Form des mit einem Zahnersatzteil zu versehenden Restzahnbereichs ermittelt, beispielsweise abgeformt wird, und
- **dass** das Zahnersatzteil aufgrund der gespeicherten Digitaldaten durch spanabhebende Bearbeitung eines Rohlings gefertigt wird,
- wobei die Fertigung anhand der gespeicherten Digitaldaten gesteuert wird.

12. Verfahren nach Anspruch 11,
**dadurch gekennzeichnet,**
**dass** eine Überwachung der Ermittlung der Form des Restzahnbereiches und eine Überwachung der Fertigung des Zahnersatzteiles mit der Speicherung der ermittelten Digitaldaten und der Steuerung der Fertigung des Zahnersatzteiles in apparativer Kombination durchgeführt werden.

13. Verfahren nach Patentanspruch **11,**
**dadurch gekennzeichnet,**
**dass** eine Steuerung des Verfahrens vor und/oder während seiner Durchführung mittels einer Eingabeeinheit durchführbar ist.

14. Verfahren nach mindestens einem der Patentansprüche **11** bis **13,**
**dadurch gekennzeichnet,**
**dass** die Vorbereitung, der Ablauf und das Ergebnis des Verfahrens visualisiert werden.

15. Verfahren nach mindestens einem der Patentansprüche **11** bis **14,**
**dadurch gekennzeichnet,**
**dass** das Verfahren mit Hilfe einer spezifischen Software durchgeführt wird.

16. Vorrichtung zur Ermittlung der Form eines mit einem Zahnersatzteil **(143)** zu versehenden Duplikats **(20)** eines Restzahnbereichs,
- mit einer Duplikats-Einspannvorrichtung **(28)** für das Duplikat **(20),** dessen Form zu ermitteln ist,
- mit einer Sensoreinrichtung **(30)** zum Ermitteln der Form des Duplikates **(20),** wobei die Duplikats-Einspannvorrichtung **(28)** und die Sensoreinrichtung **(30)** relativ zueinander bewegbar sind, und
- mit einer Speichereinheit **(112)** zum Speichern der ermittelten Daten des Duplikates **(20),**
**dadurch gekennzeichnet,**
**dass** die Vorrichtung
- eine Abschnitts-Einspannvorrichtung **(44)** für mindestens einen Duplikatsabschnitt **(42.1** bis **42.8)** besitzt, um den Duplikatsabschnitt **(42.1** bis **42.8)** während der Ermittlung seiner Form zu halten und relativ zur Sensoreinrichtung **(30)** zu bewegen,
- **dass** die Sensoreinrichtung **(30)** eine berührungsfrei wirkende Sensoreinrichtung und zum Ermitteln der Form des mindestens einen Duplikatsabschnittes **(42.1 bis 42.8)** ausgebildet ist, und
- **dass** die Speichereinheit **(112)** zum Speichern der ermittelten Daten des mindestens einen Duplikatsabschnittes **(42.1** bis **42.8)** ausgebildet ist.

17. Vorrichtung nach Patentanspruch **16,**
**dadurch gekennzeichnet,**
**dass** die berührungsfrei wirkende Sensoreinrichtung **(30)** eine mit einer digitalen Kamera **(30.2)** fest gekoppelte Lichtmusterquelle **(30.1)** ist.

18. Vorrichtung nach mindestens einem der Patentansprüche **16** bis **17,**
**dadurch gekennzeichnet,**
**dass** die Sensoreinrichtung so ausgebildet ist, dass die Form der Duplikatsabschnitte **(42.1** bis **42.8)** mit höherer Genauigkeit ermittelbar ist als die des unzerteilten Duplikates **(20).**

19. Vorrichtung nach mindestens einem der Patentansprüche **16** bis **18,**
**dadurch gekennzeichnet,**
**dass** die Abschnitts-Einspannvorrichtung **(44)** Einspanneinheiten für mehrere Duplikatsabschnitte **(42.1** bis **42.8)** besitzt.

20. Vorrichtung nach mindestens einem der Patentansprüche **16** bis **19,**
**dadurch gekennzeichnet,**
**dass** die Duplikats-Einspannvorrichtung **(28)** und die Abschnitts-Einspannvorrichtung **(28)** auf einem Tisch **(64)** angeordnet sind, welcher auf zwei orthogonalen Gleitführungen **(62)** verschiebbar gelagert ist.

21. Vorrichtung nach mindestens einem der Patentansprüche **16** bis **20,**
**dadurch gekennzeichnet,**
**dass** die Duplikats-Einspannvorrichtung **(28)** und die Einspanneinheiten der Abschnitts-Einspannvorrichtung **(44)** drehbar sind.

22. Vorrichtung nach Patentanspruch **21,**
**dadurch gekennzeichnet,**
**dass** die Duplikats-Einspannvorrichtung **(28)** und die Einspanneinheiten der Abschnitts-Einspannvorrichtung **(44)** um parallel zur Fläche des Tisches **(64)** verlaufende Achsen drehbar sind.

23. Vorrichtung nach mindestens einem der Patentansprüche **16** bis **22,**
**dadurch gekennzeichnet,**
**dass** sie Positionsmessysteme, wie beispielsweise Positionsgeber oder Massstäbe, zur Überwachung der Längslagen des Tisches **(64)** in den zwei orthogonalen Verschiebungsrichtungen und der Drehlagen der Einspannvorrichtungen **(28, 44)** besitzt.

24. Vorrichtung nach mindestens einem der Patentansprüche **16** bis **23,**
**dadurch gekennzeichnet,**
**dass** sie zur Herstellung Zahnersatzteiles im Weiteren aufweist
- eine Fertigungsmaschine **(114)** zur Fertigung des Zahnersatzteiles **(143)** aus einem Rohling **(10)** und
- eine Steuereinheit **(116)** zum Steuern der Fertigungsmaschine **(114),**
- wobei die Vorrichtung **(102)** zur Ermittlung der Form des Duplikats **(20)** des mit dem Zahnersatzteil **(143)** zu versehenden Restzahnbereichs und die Fertigungsmaschine **(114)** über eine EDV-Einrichtung gekoppelt sind, und
- in der die Formermittlungsvorrichtung **(110)** und die Fertigungsmaschine **(114)** koppelnden EDV-Einrichtung **(118)** die Speichereinheit **(112)** und die Steuereinheit **(116)** integriert sind.

25. Vorrichtung nach Patentanspruch **24,**
**dadurch gekennzeichnet,**
**dass** die EDV-Einrichtung **(118)** eine Eingabeeinheit **(120)** zur Eingabe von Daten **(VD)** aufweist, um die Herstellung des Zahnersatzteiles **(143)** zu steuern.

26. Vorrichtung nach mindestens einem der Patentansprüche **24** bis **25,**
**dadurch gekennzeichnet,**
**dass** die EDV-Anlage **(118)** eine Monitoreinheit **(122)** umfasst.

27. Vorrichtung nach mindestens einem der Patentansprüche **24** bis **26,**
**dadurch gekennzeichnet,**
**dass** die EDV-Anlage **(118)** Software **(119)** zur Herstellung von Zahnersatzteilen **(143)** enthält, welche eine Datenbank mit Daten **(BD)** aus fachübergreifendem Basiswissen und ein Programm für die Erzeugung von Daten **(FD)** für die Fertigung der Zahnersatzteile **(143)** umfasst.

28. Verwendung der Vorrichtung **(102)** nach mindestens einem der Patentansprüche **16** bis **27,**
**dadurch gekennzeichnet,**
**dass** das Zahnersatzteil **(143)** aus Metall, Keramik, Kunststoff oder einem anderen geeigneten Material hergestellt wird.

## Claims

1. Procedure for determination of the shape of a facsimile **(20)** of the region of the remaining part of a tooth to which a dental prosthesis **(143)** is to be affixed, by which the shape of the facsimile **(20)** is to be determined and the data so obtained to be stored,
**characterized in that**
- the facsimile **(20)** is divided into facsimile sections **(42.1** to **42.8);**
- the shapes of the facsimile sections **(42.1** to **42.8)** are determined separately and the data so obtained are stored; and
- the data respecting the facsimile **(20)** and the data respecting the facsimile sections **(42.1** to **42.8)** are combined.

2. Procedure according to Patent Claim **1,**
**characterized in that**
the shapes of the facsimile sections **(42.1** to **42.8)** are determined from more than one angle and the data so obtained are stored as digital data **(DD)** and combined by means of a 3D matching procedure.

3. Procedure according to Patent Claim **1,**
**characterized in that**
the shape of the facsimile **(20)** is determined from more than one angle and the data so obtained are stored as digital data **(DD)** and combined by means of a 3D matching procedure.

4. Procedure according to Patent Claims **2** and **3,**
**characterized in that**
the data obtained respecting the facsimile sections **(42.1** to **42.8)** and the facsimile **(20)** are combined.

5. Procedure according to Patent Claims **2** and **3**
**characterized in that**
determination of the shape of the facsimile **(20)** is performed with less precision than determination of the shape of each of the facsimile sections **(42.1** to **42.8).**

6. Procedure according to at least one of Patent Claims **1** to **5,**
**characterized in that**
a second facsimile **(40)** is created, after which one of the two facsimiles **(20** or **40)** is divided into the facsimile sections **(42.1** to **42.8).**

7. Procedure according to Patent Claim **6,**
**characterized in that**
the second facsimile **(40)** is produced as a secondary creation based on the first facsimile **(20).**

8. Procedure according to at least one of Patent Claims **1** to **7,**
**characterized in that**
the division of the facsimile **(40)** into facsimile sections **(42.1** to **42.8)** is performed tooth by tooth or by groups of teeth.

9. Procedure according to at least one of Patent Claims **1** to **8,**
**characterized in that**
the facsimile sections **(42.1** to **42.8)** are scanned in the same order as that which they occupied in the undivided facsimile **(40).**

10. Procedure according to at least one of Patent Claims **1** to **9,**
**characterized in that**
determination of the shape of the facsimile **(20)** and each of the facsimile sections **(42.1** to **42.4)** is carried out from more than one relative position and from each relative position in numerous parallel planes.

11. Procedure according to at least one of Patent Claims **1** to **10,**
**characterized in that**
- for creation of the facsimile, the shape of the region of the remaining part of a tooth to which a dental prosthesis is to be affixed is detected and may, for example, be moulded, and
- the dental prosthesis is made in accordance with the stored digital data by a process of removal of excess material from a blank,
- the manufacturing process being controlled by means of the stored digital data.

12. Procedure according to Patent Claim **11,**
**characterized in that**
supervision of the determination of the shape of the region of the remaining part of a tooth and supervision of the manufacture of the dental prosthesis with storage of the digital data obtained and control of the manufacture of the dental prosthesis are effected by a combination of instruments.

13. Procedure according to Patent Claim **11,**
**characterized in that**
control of the process before and/or during its execution may be effected by means of an input unit.

14. Procedure according to at least one of Patent Claims **11** to **13,**
**characterized in that**
the preparation for, execution of and result of the procedure are visually displayed.

15. Procedure according to at least one of Patent Claims **11** to **14,**
**characterized in that**
the procedure is executed by means of a specific software program.

16. Apparatus for determining the shape of a facsimile **(20)** of the region of the remaining part of a tooth to which a dental prosthesis **(143)** is to be affixed,
- with a facsimile mounting device **(28)** for the facsimile **(20)** whose shape is to be determined,
- with a sensor device **(30)** for determination of the shape of the facsimile **(20),** the facsimile mounting device **(28)** and the sensor device **(30)** being movable with respect to each other, and
- with a storage unit **(112)** for storage of the data obtained with respect to the facsimile **(20),**
**characterized in that**
the apparatus
- possesses a section mounting device **(44)** for at least one facsimile section **(42.1** to **42.8)** designed to hold the facsimile section **(42.1** to **42.8)** during determination of its shape and to move it with respect to the sensor device **(30),**
- the sensor device **(30)** is equipped with a sensor device operating without contact and is designed to determine the shape of at least one facsimile section **(42.1** to **42.8),** and
- the storage unit **(112)** is designed for storage of the data obtained with respect to at least one of the facsimile sections **(42.1** to **42.8).**

17. Apparatus according to Patent Claim **16,**
**characterized in that**
the sensor device operating without contact **(30)** consists of a standard light source **(30.1)** rigidly coupled to a digital camera **(30.2).**

18. Apparatus according to at least one of Patent Claims **16** to **17,**
**characterized in that**
the sensor device is so designed that the shape of the facsimile sections **(42.1** to **42.8)** can be determined with a higher degree of accuracy than that of the undivided facsimile **(20).**

19. Apparatus according to at least one of Patent Claims **16** to **18,**
**characterized in that**
the section mounting device **(44)** possesses mounting units for several facsimile sections **(42.1** to **42.8).**

20. Apparatus according to at least one of Patent Claims **16** to **19,**
**characterized in that**
the facsimile mounting device **(28)** and the section mounting device **(44)** are set up on a table **(64)** which is fitted so that it can slide on two orthogonally positioned guide rails **(62).**

21. Apparatus according to at least one of Patent Claims **16** to **20,**
**characterized in that**
the facsimile mounting device **(28)** and the mounting units of the section mounting device **(44)** can be rotated.

22. Apparatus according to Patent Claim **21,**
**characterized in that**
the facsimile mounting device **(28)** and the mounting units of the section mounting device **(44)** can pivot about axles running in a direction parallel to the surface of the table **(64).**

23. Apparatus according to at least one of Patent Claims **16** to **22,**
**characterized in that**
it includes a system for the measurement of position, as for example position indicators or graduated scales, designed to monitor the longitudinal position of the table **(64)** in the two orthogonal directions of sliding movement and the angular position of the mounting devices **(28, 44).**

24. Apparatus according to at least one of Patent Claims **16** to **23,**
**characterized in that**
for the manufacture of the dental prosthesis it is also equipped with
- a processing machine **(114)** to make the dental prosthetic part **(143)** from a blank **(10)** and
- a control unit **(116)** to control the processing machine **(114),**
- the apparatus **(102)** for determination of the shape of a facsimile **(20)** of the region of the remaining part of a tooth to which a dental prosthesis **(143)** is to be affixed and the processing machine **(114)** being coupled via an electronic data processing device, and
- the electronic data processing device **(118)** coupling the device for determining the shape **(110)** and the processing machine **(114)** incorporates the storage unit **(112)** and the control unit **(116).**

25. Apparatus according to Patent Claim **24,**
**characterized in that**
the electronic data processing device **(118)** is equipped with an input unit **(120)** for the input of data **(VD)** to control the manufacture of the dental prosthesis **(143).**

26. Apparatus according to at least one of Patent Claims **24** to **25,**
**characterized in that**
the electronic data processing device **(118)** incorporates a monitor unit **(122).**

27. Apparatus according to at least one of Patent Claims **24** to **26,**
**characterized in that**
the electronic data processing device **(118)** contains software **(119)** for the manufacture of dental prostheses **(143),** including a database of data **(BD)** deriving from the fundamental knowledge of the domain and a program for the generation of data **(FD)** for the production of dental prostheses **(143).**

28. Utilization of the apparatus **(102)** according to at least one of Patent Claims **16** to **27,**
**characterized in that**
the dental prosthesis **(143)** is made from metal, ceramic, plastic or other suitable material.

## Revendications

1. Procédé de détermination de la forme d'une réplique **(20)** d'un chicot à équiper d'un élément de prothèse dentaire **(143),** dans lequel la forme de la réplique **(20)** est déterminée et les données alors déterminées sont mémorisées,
**caractérisé**
- **en ce que** la réplique **(20)** est divisée en segments de réplique **(42.1** à **42.8);**
- **en ce que** la forme des segments de réplique **(42.1** à **42.8)** est déterminée séparément et les données déterminées à cet égard sont mémorisées ; et
- **en ce que** les données de la réplique **(20)** et les données des segments de réplique **(42.1** à **42.8)** sont rassemblées.

2. Procédé selon la revendication 1, **caractérisé en ce que** la forme des segments de réplique **(42.1** à **42.8)** est détectée selon différents angles et les données déterminées sont mémorisées en tant que données numériques **(DN)** et rassemblées au moyen d'un procédé de mise en correspondance 3D.

3. Procédé selon la revendication **1, caractérisé en ce que** la forme de la réplique **(20)** est détectée selon différents angles et les données déterminées sont mémorisées en tant que données numériques **(DN)** et rassemblées au moyen d'un procédé de mise en correspondance 3D.

4. Procédé selon la revendication **2** et **3, caractérisé en ce que** les données déterminées des segments de réplique **(42.1** à **42.8)** et de la réplique **(20)** sont rassemblées.

5. Procédé selon les revendications **2** et **3, caractérisé en ce que** la détermination de la forme de la réplique **(20)** se fait avec une moindre précision que la détermination de la forme de chaque segment de réplique **(42.1** à **42.8).**

6. Procédé selon au moins l'une des revendications **1** à **5, caractérisé en ce qu**'on fabrique une seconde réplique **(40),** suite à quoi une des deux répliques **(20** ou **40)** est divisée en les segments de réplique **(42.1** à **42.8).**

7. Procédé selon la revendication **6, caractérisé en ce que** la seconde réplique **(40)** est fabriquée comme réplique secondaire sur la base de la première réplique **(20).**

8. Procédé selon au moins l'une des revendications **1** à **7, caractérisé en ce que** la division de la réplique **(40)** en segments de répliques **(42.1** à **42.8)** se fait par dent ou par groupe de dents.

9. Procédé selon au moins l'une des revendications **1** à **8, caractérisé en ce que** les segments de réplique **(42.1** à **42.8)** sont analysés dans l'ordre selon lequel ils sont pris dans la réplique non divisée **(40).**

10. Procédé selon au moins l'une des revendications **1** à **9, caractérisé en ce que** la détermination de la forme de la réplique **(20)** et de chacun des segments de réplique **(42.1** à **42.4)** se fait selon plusieurs positions angulaires et, dans chaque position angulaire, selon plusieurs plans parallèles.

11. Procédé selon au moins l'une des revendications **1** à **10, caractérisé en ce que,**
- pour fabriquer la réplique, on détermine la forme du chicot à pourvoir d'un élément de prothèse dentaire, par exemple par modelage, et
- **en ce que** l'élément de prothèse dentaire, sur la base des données numériques mémorisées, est fabriqué par usinage par enlèvement de copeaux d'une ébauche,
- la fabrication étant commandée au moyen des données numériques mémorisées.

12. Procédé selon la revendication **11, caractérisé en ce qu**'une surveillance de la détermination de la forme du chicot et une surveillance de la fabrication de l'élément de prothèse dentaire sont réalisées avec la mémorisation des données numériques déterminées et avec la commande de la fabrication de l'élément de prothèse dentaire dans une combinaison d'appareils.

13. Procédé selon la revendication **11, caractérisé en ce qu'**une commande du procédé peut être réalisée avant et/ou pendant son exécution au moyen d'une unité d'entrée.

14. Procédé selon au moins l'une des revendications **11** à **13, caractérisé en ce que** la préparation, le déroulement et le résultat du procédé sont visualisés.

15. Procédé selon au moins l'une des revendications **11** à **14, caractérisé en ce que** le procédé est réalisé à l'aide d'un logiciel spécifique.

16. Dispositif de détermination de la forme d'une réplique **(20)** d'un chicot à pourvoir d'un élément de prothèse dentaire **(143),**
- comprenant un dispositif d'encastrement de réplique **(28)** pour la réplique **(20),** dont la forme doit être déterminée,
- comprenant un dispositif de capteur **(30)** destiné à déterminer la forme de la réplique **(20),** où le dispositif d'encastrement de réplique **(28)** et le dispositif de capteur **(30)** peuvent être déplacés l'un par rapport à l'autre, et
- comprenant une unité de mémorisation **(112)** destinée à mémoriser les données déterminées de la réplique **(20),**
**caractérisé en ce que** le dispositif possède
- un dispositif d'encastrement de segment **(44)** pour au moins un segment de réplique **(42.1** à **42.8),** pour maintenir le segment de réplique **(42.1** à **42.8)** pendant la détermination de sa forme et le déplacer par rapport au dispositif de capteur **(30),**
- **en ce que** le dispositif de capteur **(30)** est formé par un dispositif de capteur agissant sans contact et servant à déterminer la forme d'au moins un segment de réplique **(42.1** à **42.8),** et
- **en ce que** l'unité de mémorisation **(112)** est conçue pour mémoriser les données déterminées d'au moins un segment de réplique **(42.1** à **42.8).**

17. Dispositif selon la revendication **16, caractérisé en ce que** le dispositif de capteur **(30)** agissant sans contact est une source lumineuse modèle **(30.1)** reliée par accouplement à une caméra numérique **(30.2).**

18. Dispositif selon au moins l'une des revendications **16** à **17, caractérisé en ce que** le dispositif de capteur est conçu de façon telle que la forme des segments de réplique **(42.1** à **42.8)** peut être déterminée avec une précision plus élevée que celle de la réplique non divisée **(20).**

19. Dispositif selon au moins l'une des revendications **16** à **18, caractérisé en ce que** le dispositif d'encastrement de segment **(44)** possède des unités d'encastrement pour plusieurs segments de réplique **(42.1** à **42.8).**

20. Dispositif selon au moins l'une des revendications **16** à **19, caractérisé en ce que** le dispositif d'encastrement de réplique **(28)** et le dispositif d'encastrement de segments **(28)** sont disposés sur une table **(64)** qui est logée de façon mobile sur deux glissières **(62)** orthogonales.

21. Dispositif selon au moins l'une des revendications **16** à **20, caractérisé en ce que** le dispositif d'encastrement de réplique **(28)** et les unités d'encastrement du dispositif d'encastrement de segment **(44)** peuvent pivoter.

22. Dispositif selon la revendication **21, caractérisé en ce que** le dispositif d'encastrement de réplique **(28)** et les unités d'encastrement du dispositif d'encastrement de segment **(44)** peuvent pivoter autour d'axes s'étendant parallèlement à la surface de la table **(64).**

23. Dispositif selon au moins l'une des revendications **16** à **22, caractérisé en ce qu**'il possède des systèmes de mesure de position, comme par exemple des indicateurs de position ou des règles graduées, pour surveiller les positions longitudinales de la table **(64)** dans les deux directions de déplacement orthogonales et les positions de pivotement des dispositifs d'encastrement **(28, 44).**

24. Dispositif selon au moins l'une des revendications **16** à **23, caractérisé en ce qu**'il présente en outre, pour fabriquer l'élément de prothèse dentaire,
- une machine de fabrication **(114)** destinée à fabriquer l'élément de prothèse dentaire **(143)** à partir d'une ébauche **(10)** et
- une unité de commande **(116)** destinée à commander la machine de fabrication **(114),**
- le dispositif **(102)** de détermination de la forme de la réplique **(20)** du chicot, à pourvoir de l'élément de prothèse dentaire **(143),** et la machine de fabrication **(114)** étant couplés par un dispositif de traitement informatique, et
- l'unité de mémorisation **(112)** et l'unité de commande **(116)** étant intégrées dans le dispositif de traitement informatique **(118)** couplant le dispositif de détermination de la forme **(110)** et la machine de fabrication **(114).**

25. Dispositif selon la revendication **24, caractérisé en ce que** le dispositif de traitement informatique **(118)** présente une unité d'entrée **(120)** permettant l'entrée de données **(VD)** pour commander la fabrication de l'élément de prothèse dentaire **(143).**

26. Dispositif selon au moins l'une des revendications **24** à **25, caractérisé en ce que** le dispositif de traitement informatique **(118)** possède un moniteur **(122).**

27. Dispositif selon au moins l'une des revendications **24** à **26, caractérisé en ce que** l'unité de traitement informatique **(118)** contient un logiciel **(119)** de fabrication d'éléments de prothèse dentaire **(143),** qui comprend une banque de données présentant des données **(BD)** concernant des connaissances pluridisciplinaires et un programme de génération des données **(FD)** pour la fabrication des éléments de prothèse dentaire.

28. Utilisation du dispositif **(102)** selon au moins l'une des revendications **16** à **27, caractérisée en ce que** l'élément de prothèse dentaire **(143)** est fabriqué en métal, en céramique, en matériau synthétique ou dans un autre matériau approprié.
